# EUROPÄISCHE PATENTSCHRIFT

(11) **EP 0 832 630 B1**
(45) Veröffentlichungstag und Bekanntmachung des Hinweises auf die Patenterteilung: **02.05.2002**
(21) Anmeldenummer: 97111489.7
(22) Anmeldetag: 08.07.1997
(51) Int. Cl.: A61F 13/58

(54) **Verschlussband für eine Höschenwindel**
Diaper fastening tape
Ruban de fermeture pour couche

(30) Priorität: 25.09.1996 DE 29616711 U
(43) Veröffentlichungstag der Anmeldung: 01.04.1998
(73) Patentinhaber: Koester GmbH & Co. KG, 96146 Altendorf (DE)
(72) Erfinder: Bräunig, Werner, 97514 Oberaurach (DE); Lanz, Jörg, 91052 Erlangen (DE)
(74) Vertreter: Schneck, Herbert, Dipl.-Phys., Dr.

(56) Entgegenhaltungen:
- EP-A- 0 249 073
- WO-A-96/21413
- US-A- 4 522 853

## Beschreibung

Die Erfindung betrifft ein Verschlußband für eine Höschenwindel mit den im Oberbegriff des Anspruches 1 angegebenen Merkmalen.

Ein derartiges Verschlußband ist aus der US-A-4 522 853 bekannt und dient üblicherweise zur Klebeverbindung des Rückenteils der Windel mit dem durch den Schrittbereich des Kindes hochgeklappten Windel-Vorderteil. Je ein solches streifenförmiges Verschlußband liegt dabei im linken und rechten Hüftbereich der Höschenwindel.

Der gattungsgemäße Verschlußstreifen ist mit einem mit dem rückwärtigen Windelteil vorzugsweise dauerhaft verbundenen, praktisch unelastischen Trägerabschnitt und einem im Gebrauchszustand mit dem vorderen Windelteil verbindbaren, praktisch unelastischen Trägerabschnitt versehen. Zwischen diesen beiden Trägerabschnitten liegt ein elastischer Zwischenabschnitt, der einerseits den Tragekomfort bei gleichzeitig dichter Anlage des oberen Windelrandes an den Körper erhöht und welcher es andererseits ermöglicht, den oberen Windelrand ohne Lösen der Klebeverschlüsse etwas vom Körper des Kindes abzuheben, um den Zustand des Windelinneren prüfen zu können.

Bei dem elastischen Verschlußband gemäß US-A-4 522 853 sind sowohl die beiden Trägerabschnitte, als auch der Zwischenabschnitt aus extrudierfähigen Materialien gefertigt, wobei der nicht mit Klebstoff Versehene Teil der beiden Trägerabschnitte mit dem Zwischenstück thermisch durch eine Art Verschweißung mit einem Schweißmaterial verbunden ist. Dies ist herstellungstechnisch nicht unkompliziert und insbesondere kritisch hinsichtlich der Übergangsbereiche zwischen den unterschiedlichen Extrusionsmaterialien und der dort erzielbaren Stabilität.

Davon ausgehend liegt der Erfindung die Aufgabe zugrunde, ein Verschlußband der gattungsgemäßen Art so weiterzubilden, daß unter Aufrechterhaltung der Vorteile des elastischen Zwischenabschnittes eine einfache Herstellbarkeit und besonders stabile Verbindung zwischen den Trägerabschnitten und dem Zwischenabschnitt erzielt wird.

Diese Aufgabe wird durch die im Kennzeichnungsteil des Anspruches 1 angegebenen Merkmale gelöst. Demnach sind die beiden Trägerabschnitte aus Vliesfasern zumindest in ihrem Verbindungsbereich mit dem Zwischenabschnitt mit einer offenen Oberfläche mit von außen zugänglichen Zwischenräumen Zwischen den Vliesfasern versehen. Der Zwischenabschnitt besteht aus thermoplastischem, elastischen Material, das durch Eindringen in geschmolzenem Zustand in die Oberflächenöffnungen der Trägerabschnitte und anschließendes Erstarren mechanisch verankert und somit auf die Trägerabschnitte aufextrudiert ist ist. Diese mechanische Verbindung ist besonders innig und damit stabil, gleichzeitig jedoch sehr einfach herzustellen. Die durch den elastischen Zwischenabschnitt grundsätzlich erzielbaren Vorteile im Zusammenhang mit den Trage- und Gebrauchseigenschaften der Windel bleiben dabei voll erhalten. Darüber hinaus wurde überraschenderweise festgestellt, daß durch die Verwendung des erfindungsgemäßen Verschlußbandes eine erhebliche Verbesserung der Windel-Paßform erzielbar ist, was wiederum zu einer deutlichen Erhöhung der Auslauffestigkeit der Windel führt.

Weitere Merkmale, Einzelheiten und Vorteile der Erfindung sind den Unteransprüchen und der nachfolgenden Beschreibung entnehmbar, in der ein Ausführungsbeispiel des Erfindungsgegenstandes näher erläutert wird. Es zeigen:
- Fig. 1: eine schematische Draufsicht auf ein Verschlußband, und
- Fig. 2: einen höchst schematischen Schnitt durch des Verschlußband entlang der Schnittlinie II-II nach Fig. 1.

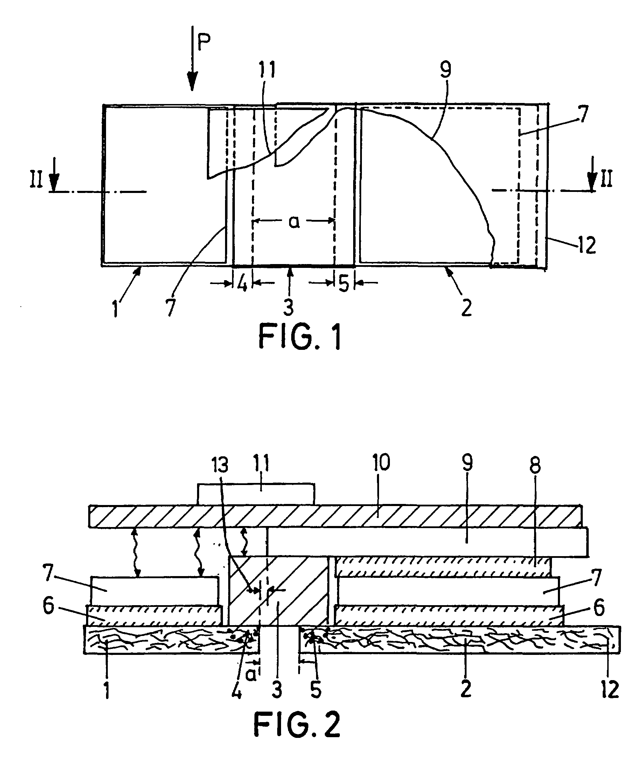

Das in Fig. 1 in Draufsicht dargestellte und in Fig. 2 in einem höchst schematischen Schnitt gezeigte Verschlußband besteht aus einem ersten Trägerabschnitt 1 aus praktisch unelastischem, thermofixierten Polypropylen-Vlies. Der zweite Trägerabschnitt 2 besteht aus dem gleichen Material, das sich durch eine offene Oberfläche und von außen zugängliche Zwischenräume zwischen den Vliesfasern auszeichnet.

Beide Trägerabschnitte 1, 2, die in Draufsicht (Fig. 1) rechteckig ausgebildet sind, sind durch einen streifenförmigen Zwischenabschnitt 3 miteinander verbunden, der aus thermoplastischem, elastischen Material besteht. Dabei handelt es sich um ein modifiziertes Styrol-Ethylen-Buthylen-Styrol-Block-Copolymerisat, das eine Bruchdehnung von 300 % und ein Elastizitätsmodul von vorzugsweise 1,6 N/mm² aufweist. Als geeigneter Bereich für das Elastizitätsmodul sind Werte zwischen 0,5 N/mm² und 3,5 N/mm² anzugeben.

Die Herstellung dieser Verbindung erfolgt dadurch, daß auf zwei in Produktionsrichtung P gemäß Fig. 1 in dem gezeigten Abstand a laufende Vliesstreifen der Zwischenabschnitt 3 aufextrudiert wird. Dabei ist dessen thermoplastisches Material geschmolzen und kann in die Zwischenräume des Vliesmaterials über dessen offene Oberfläche eindringen. Durch das Erstarren des Thermoplastmaterials wird eine innige, mechanische Verbindung zwischen dem Streifen des Zwischenabschnittes 3 innerhalb der Verbindungsbereiche 4, 5 mit den Trägerabschnitten 1, 2 erreicht.

Auf die beiden Trägerabschnitte 1, 2 ist über eine Permanentkleberschicht 6 jeweils ein Folienstreifen 7 aufgebracht, mit dem die Trägerabschnitte 1, 2 verstärkt und auf dieser Seite mit einer glatten Oberfläche versehen werden. Auf den Folienstreifen 7 des Trägerabschnittes 2 wird über eine wiederablösbare Kleberschicht 8 ein Ablösestreifen 9 - ein sogenanntes "Release-Tape" - aufgebracht, das teilweise den Zwischenabschnitt 3 übergreift.

Der gesamte bisherige Aufbau des Verschlußbandes ist mit einer Permanentkleberschicht 10 abgedeckt, die direkt auf dem Folienstreifen 7 des Trägerabschnittes 1 (wie durch gewellte Pfeile in Fig. 2 angedeutet ist), der freiliegenden Oberfläche des Zwischenabschnittes 3 und dem Ablösestreifen 9 verläuft. Schließlich ist auf der Permanentkleberschicht 10 noch ein zentraler Streifen 11 vorgesehen, der sich vom Ablösestreifen 9 über den Zwischenabschnitt 3 zum Folienstreifen 7 erstreckt. Damit wird eine im Zustand gemäß Fig. 2 unelastische Verbindung zwischen dem Ablösestreifen 9 und dem Folienstreifen 7 geschaffen. Dies erleichtert die maschinelle Applikation des Verschlußbandes, da der elastische Zwischenabschnitt 3 auf diese Weise inaktiviert ist. Im übrigen bildet der Abstandsspalt 13 zwischen den einander zugewandten Rändern des Trägerabschnittes 1 und des Ablösestreifens 9 eine Art Sollknickstelle, wodurch sich das Verschlußband beim später noch genauer zu beschreibenden Applizieren auf das Rückenteil der Windel im Bereich dieser Sollknickstelle leicht falten läßt. Dies kommt ebenfalls der maschinellen Applizierbarkeit zugute.

Schließlich ist darauf hinzuweisen, daß statt des Ablösestreifens 9 und der wiederablösbaren Kleberschicht 8 eine übliche lösbare Verbindung aus Hakenmaterial - einem sogenannten "Klettband" - verwendet werden kann.

Zur Fertigung des Verschlußbandes ist festzuhalten, daß der in Fig. 2 umrissene Lagenaufbau durch eine Endlosfertigung aus den entsprechenden Lagen und Schichten in Produktionsrichtung P (Fig. 1) hergestellt wird, so daß Rollenware mit dem in Fig. 2 gezeigten Querschnitt entsteht. Dieser wird zur Zwischenlagerung aufgerollt, wobei der Streifen 11 verhindert, daß die Permanentkleberschicht 10 sich mit dem Zwischenabschnitt 3 der nächsten Lage verklebt. Ein Abdecken der Permanentkleberschicht 10 in den sich in den Trägerabschnitten 1, 2 überdeckenden seitlichen Bereichen ist nicht notwendig, da der Klebstoff der Schicht 10 nicht oder nur in einem unerheblichen Maße an dem Vliesmaterial der Trägerabschnitte 1, 2 haftet.

Von der so angelieferten Rolle werden quer zur Produktionsrichtung P die Verschlußbänder abgelängt und auf die Windel aufgebracht. Dabei wird der Trägerabschnitt 1 mit der Permanentkleberschicht 10 an der Außenseite des Rückenteils der Windel befestigt und der Trägerabschnitt 2 um den Vorderrand des Rückenteils herumgefaltet, so daß dieser Teil mittels der Permanentkleberschicht 10 an der Innenseite des Rückenteils befestigt wird. Dieses Umfalten wird durch die oben erörterte Sollknickstelle zwischen Folienstreifen 7 und Ablösestreifen 9 unterstützt. Bei der Anwendung der Windel wird die vom Trägerabschnitt 2 gebildete Grifflasche 12 erfaßt und der Trägerabschnitt 2 vom Rückenteil der Windel weggezogen, so daß sich die wiederablösbare Kleberschicht 8 vom Ablösestreifen 9 löst und mit dieser der Trägerabschnitt 2 auf der Außenseite des Windelvorderteils fixiert werden kann. Beim Ablösevorgang kommt der elastische Zwischenabschnitt auch vom Streifen 11 frei und ist somit innerhalb seiner Elastizitätsgrenzen dehnbar. Beim Fixiervorgang kann der Zwischenabschnitt 3 gedehnt werden, um einen optimalen Sitz zu erreichen. Wie bereits erwähnt, ist es möglich, ein Verschlußband der in Betracht stehenden Art statt als Klebeband auch als mechanisches Verschlußsystem auszubilden, d. h. statt der Klebeflächen Haken- bzw. Ösenmaterial aufzubringen. Das Aufbringen des bandförmigen Haken- bzw. Schlaufinaterials kann beispielsweise durch Aufkaschieren auf die Klebeschicht 8 vorgenommen werden.

## Patentansprüche

1. Verschlußband für eine Höschenwindel mit
- einem mit einem rückwärtigen Windelteil vorzugsweise dauerhaft verbindbaren, praktisch unelastischen Trägerabschnitt (1) aus Vliesfasern,
- einem im Gebrauchszustand mit einem vorderen Windelteil vorzugsweise lösbar verbindbaren, unelastischen Trägerabschnitt (2), und
- einem elastischen Zwischenabschnitt (3) zwischen den beiden Trägerabschnitten (1, 2),
- einem Zwischenabschnitt aus thermoplastischem, elastischen Material
**dadurch gekennzeichnet,**
- **daß** beide Trägerabschnitte (1, 2) zumindest in ihrem Verbindungsbereich (4, 5) mit dem Zwischenabschitt (3) mit einer offenen Oberfläche mit von außen zugänglichen Zwischenräumen zwischen den Vliesfasern versehen sind, und
- **daß** durch Eindringen des Materials des Zwischen abschnittes im erweichten Zustand in die Oberflächenöffnungen der Trägerabschnitte (1, 2) der Zwischenabschnitt (3) in den Trägerabschnitten (1, 2) mechanisch verankert ist, und
- **daß** der Zwischenabschnitt (3) auf die Trägerabschnitte (1, 2) aufextrudiert ist.

2. Verschlußband nach Anspruch 1, **dadurch gekennzeichnet, daß** die Trägerabschnitte aus einem thermofixierten Polypropylen-Vliesmaterial bestehen.

3. Verschlußband nach einem der Ansprüche 1 oder 2, **dadurch gekennzeichnet, daß** die Trägerabschnitte (1, 2) außerhalb ihres Verbindungsbereiches (4, 5) mit einer Abdecklage (7) mit geschlossener Oberfläche versehen sind.

4. Verschlußband nach einem der Ansprüche 1 bis 3, **dadurch gekennzeichnet, daß** das elastische Material des Zwischenabschnittes ein modifiziertes Styrol-Ethylen-Buthylen-Styrol-Block-Copolymerisat ist.

5. Verschlußband nach einem der Ansprüche 1 bis 4, **dadurch gekennzeichnet, daß** das elastische Material des Zwischenabschnittes (3) eine Bruchdehnung von mindestens 250 % und ein Elastizitätsmodul von 0,5 N/mm² bis 3,5 N/mm², vorzugsweise von 1,6 N/mm², aufweist.

6. Verschlußband nach einem der Ansprüche 1 bis 5, **dadurch gekennzeichnet, daß** der vordere unelastische Trägerabschnitt (2) mit dem vorderen Windelteil mittels einer auf den Trägerabschnitt (2) aufgebrachten Beschichtung aus einem wiederablösbaren Kleber (8) verbindbar ist.

7. Verschlußband nach einem der Ansprüche 1 bis 5, **dadurch gekennzeichnet, daß** der vordere unelastische Trägerabschnitt (2) mit dem vorderen Windelteil mittels einer lösbaren Verbindung aus Hakenmaterial verbindbar ist.

## Claims

1. A fastening tape for diaper pants, comprising
- a virtually inelastic support section (1) of non-woven fibers, which is preferably durably linkable to a rear diaper portion;
- an inelastic support section (2), which, in use, is preferably releasably linkable to a front diaper portion; and
- an elastic intermediate section (3) between the two support sections (1, 2);
- an intermediate section of thermoplastic, elastic material;
**characterized**
- **in that** both support sections (1, 2), at least in the area (4, 5) of linkage to the intermediate section (3), are provided with an open surface with externally accessible interspaces between the non-woven fibers; and
- in that the intermediate section (3) is mechanically anchored in the support sections (1, 2) by the intermediate section material, when soaked, penetrating into the surface openings of the support sections (1, 2); and
- in that the intermediate section (3) is applied by extrusion on the support sections (1, 2).

2. A fastening tape according to claim 1, **characterized in that** the support sections consist of heat-set polypropylene non-wovens.

3. A fastening tape according to one of claims 1 or 2, **characterized in that** the support sections (1, 2), outside their area of linkage (4, 5), are provided with a cover layer (7) of a closed surface.

4. A fastening tape according to one of claims 1 to 3, **characterized in that** the elastic material of the intermediate section is a modified styrene-ethylene-butylene-styrene-block copolymer.

5. A fastening tape according to one of claims 1 to 4, **characterized in that** the elastic material of the intermediate section (3) has an elongation at rupture of at least 250 % and a modulus of elasticity of 0.5 N/mm² to 3.5 N/mm², preferably 1.6 N/mm².

6. A fastening tape according to one of claims 1 to 5, **characterized in that** the inelastic front support section is linkable to the front diaper portion by means of a coating of a detachable adhesive (8) applied on the support section (2).

7. A fastening tape according to one of claims 1 to 5, **characterized in that** the inelastic front support section (2) is linkable to the front diaper portion by means of a releasable connection of Velcro material.

## Revendications

1. Ruban de fermeture pour une couche-culotte, comprenant :
- un tronçon porteur (1) en nappe de fibres, pratiquement inélastique et susceptible d'être relié, de préférence durablement, avec une partie postérieure de la couche,
- un tronçon porteur (2) inélastique et relié à l'état d'utilisation à une partie antérieure de la couche, de préférence de manière détachable, et
- un tronçon intermédiaire élastique (3) entre les deux tronçons porteurs (1,2),
- un tronçon intermédiaire en matériau thermoplastique élastique,
**caractérisé en ce que**
- les deux tronçons porteurs (1, 2) sont pourvus, au moins dans leur zone de liaison (4, 5) avec le tronçon intermédiaire (3), d'une surface ouverte avec des espaces intermédiaires accessibles de l'extérieur entre les nappes de fibres,
- par pénétration du matériau du tronçon intermédiaire, à l'état ramolli, dans les ouvertures de surface des tronçons porteurs (1, 2), le tronçon intermédiaire (3) est ancré de manière mécanique dans les tronçons porteurs (1, 2), et
- le tronçon intermédiaire (3) est extrudé sur les tronçons porteurs (1,2).

2. Ruban de fermeture selon la revendication 1, **caractérisé en ce que** les tronçons porteurs sont réalisés en un matériau en forme de nappe, constitué de polypropylène thermofixé.

3. Ruban de fermeture selon l'une ou l'autre des revendications 1 et 2, **caractérisé en ce que** les tronçons porteurs (1, 2) sont pourvus d'une couche de couverture (7) à surface fermée à l'extérieur de leur zone de liaison (4, 5).

4. Ruban de fermeture selon l'une des revendications 1 à 3, **caractérisé en ce que** le matériau élastique du tronçon intermédiaire est un copolymère en masse modifié styrène-éthylène-butylène-styrène.

5. Ruban de fermeture selon l'une des revendications 1 à 4, **caractérisé en ce que** le matériau élastique du tronçon intermédiaire (3) présente un allongement à la rupture d'au moins 250% et un module d'élasticité de 0,5 N/mm² à 3,5 N/mm², de préférence de 1,6 N/mm².

6. Ruban de fermeture selon l'une des revendications 1 à 5, **caractérisé en ce que** le tronçon porteur antérieur inélastique (2) est susceptible d'être relié à la partie antérieure de la couche au moyen d'un revêtement, appliqué sur le tronçon porteur (2) d'une colle (8) à nouveau séparable.

7. Ruban de fermeture selon l'une des revendications 1 à 5, **caractérisé en ce que** le tronçon porteur antérieur inélastique (2) est susceptible d'être relié à la partie antérieure de la couche au moyen d'une liaison détachable en matériau à crochets.
